Europäisches Patentamt
European Patent Office
Office européen des brevets

(19)

(11) Veröffentlichungsnummer: **0 286 803 A2**

# EUROPÄISCHE PATENTANMELDUNG

(12)

(21) Anmeldenummer: **88102601.7**

(51) Int. Cl.4: **A61L 15/00**

(22) Anmeldetag: **23.02.88**

(30) Priorität: **11.04.87 DE 3712445**

(43) Veröffentlichungstag der Anmeldung: **19.10.88 Patentblatt 88/42**

(84) Benannte Vertragsstaaten: **AT BE CH DE ES FR IT LI LU NL**

(71) Anmelder: **Vereinigte Papierwerke AG**
**Schoppershofstrasse 80**
**D-8500 Nürnberg(DE)**

(72) Erfinder: **Kubicki, Jörn, Dr.**
**Siebenbürger Strasse 39**
**D-8500 Nürnberg(DE)**
Erfinder: **Petranyi, Pal, Dr.**
**Schweriner Strasse 15**
**D-8500 Nürnberg(DE)**

(74) Vertreter: **Pohl, Hans Ludwig**
**Hefnersplatz 3**
**D-8500 Nürnberg 11(DE)**

## (54) Hygienisches Zellstoffprodukt mit verbesserter Hautverträglichkeit.

(57) Es wird ein hygienisches Zellstoffprodukt, beispielsweise eine Windel, Krankenunterlage oder Damenbinde beschrieben, bei der wenigstens eine Schicht des Produktes mit partiell neutralisierter Zitronensäure der Zusammensetzung

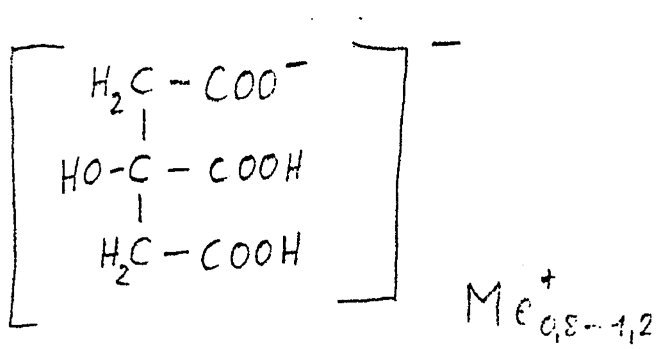

imprägniert ist, worin $Me^+$ ein Natrium-, Kalium-oder Ammonium-Ion bedeutet. Die imprägnierte Schicht soll 1,0 - 3,0 Gewichtsprozent partiell neutralisierte Zitronensäure enthalten. Im Saugkörper können zusätzlich noch Quellstoffe vorhanden sein. Desgleichen wird ein Verfahren zum Herstellen dieses Produktes beschrieben.

EP 0 286 803 A2

## Hygienisches Zellstoffprodukt mit verbesserter Hautverträglichkeit

Die Erfindung betrifft ein hygienisches Zellstoffprodukt, beispielsweise eine Windel, eine Krankenunterlage, eine Damenbinde oder eine Slipeinlage, sämtlich mit verbesserter Hautverträglichkeit. Produkte dieser Art bestehen aus einem Saugkissen, welches im wesentlichen aus Zellstoff-Flocken, gegebenenfalls unter Zusatz von Quellstoffen aufgebaut ist. Das Saugkissen ist meist mit einer flüssigkeitsdurchlässigen oberen Abdeckung aus Vliesstoff sowie gegebenenfalls einer flüssigkeitsdichten unteren Abdeckung oder Einlage ausgestattet. Die Abdeckung hat den Zweck, die Flocken des Saugkissens zusmmenzuhalten. Auf die Abdeckung kann verzichtet werden, wenn die Flocken mit geeigneten Kunststoffen, z. B. thermoplastischen Fasern oder Partikeln untereinander verbunden werden. Hygienische Zellstoffprodukte dieser Art sind allgemein bekannt.

Eine besondere Schwierigkeit beim bestimmungsgemäßen Gebrauch dieser Produkte besteht darin, daß diese nach Benetzung mit Körperflüssigkeit einen idealen Nährboden für Mikroorganismen darstellen. Das Wachstum dieser Organismen wird noch dadurch begünstigt, daß sich die Produkte in unmittelbarem Körperkontakt befinden und folglich zumindest an ihrer Oberfläche auf ideale Wachstumstemperatur erwärmt werden. Die Organismen, insbesondere Bakterien und Pilze, spalten dabei die in der Körperflüssigkeit vorhandenen Eiweiß-Stoffe sowie Harnstoff und setzen unter Anhebung des PH-Wertes aggressive Stoffe frei, unter denen insbesondere Ammoniak zu nennen ist. Diese Stoffe, die naturgemäß ebenfalls im unmittelbaren Kontakt mit der Haut des Trägers sind, führen zu Irritationen, die je nach den herrschenden Umständen sowie der Anfälligkeit des Trägers zu Hautrötungen aber auch zu Entzündungen und Infektionen führen können.

Zur Unterdrückung dieser Erscheinung ist es bekannt, baktericid, fungicid und bakteriostatisch wirkende Stoffe in die hydgienischen Zellstoffprodukte einzuarbeiten. So geht beispeilsweise aus der DE-OS 20 27 809 hervor, hygienische Zellstoffartikel, wie beispielsweise Zellstoff-Windeln, mit Zitronensäure zu imprägnieren, wobei Zitronensäuremengen von wenigstens 1,5 g/m$^2$ des Produktes angewandt werden sollen. Anstelle von Zitronensäure kann auch Apfelsäure, Weinsäure, Maleinsäure, Fumarsäure, Malonsäure oder Bernsteinsäure eingesetzt werden. Die genannten Säuren sollen dabei bewirken, daß das freiwerdenede Ammoniak sogleich absorbiert und auf diese Weise unschädlich gemacht wird. Was durch diese vorbekannten Produkte ausgenutzt werden soll, ist also lediglich die saure Natur der genannten Stoffe, wobei, wie die Beispiele zeigen, die Natur des vorhandenen Anions keine Rolle spielt.

Aus der europäischen Offenlegungsschrift 097 995 geht ein Verfahren zum antimikrobiellen Behandeln von Vliesstoffen, Papier oder dergl. hervor, welches darin besteht, daß die Stoffe zunächst mit einem Bindemittelsystem behandelt werden, welches ein Salz einer antimikrobiell wirkenden Monocarbonsäure enthält. Als Monocarbonsäuren sind nichtaromatische Säuren mit 3 bis 12 Kohlenstoffatomen genannt, z. B. Natrium-Oktanat. Außer diesen antimikrobiell wirkenden Stoffen soll dann ferner noch ein Protonen-Donator zugesetzt werden, der die Aufgabe hat, den PH-Wert zu erniedrigen. Als Protonen-Donator wird Zitronensäure genannt, wobei betont wird, daß diese Säure selbst keine antimikrobielle Wirkung entfaltet.

Die vorbekannten hygienischen Zellstoffprodukte weisen den Nachteil auf, daß sie entweder (DE-OS 20 27 809) eine zu hohe Konzentration der angewandten Säure benötigen und deshalb von sich aus zu Hautirritationen führen können, oder daß sie (EU-OS 097 995) zu Produkten mit stark herabgesetztem Saugvermögen führen und aus diesem Grunde nicht allgemein anwendbar sind.

Überraschenderweise wurde nun beobachtet, daß partiell neutralisierte Zitronensäure nach Absorption auf Zellstoff-Flocken oder -Fasern von sich aus sowohl bakterizide wie auch fungizide Wirkung zeigt. Diese Wirkung ist optimiert, wenn die partiell neutralisierte Zitronensäure folgende Zusammensetzung aufweist:

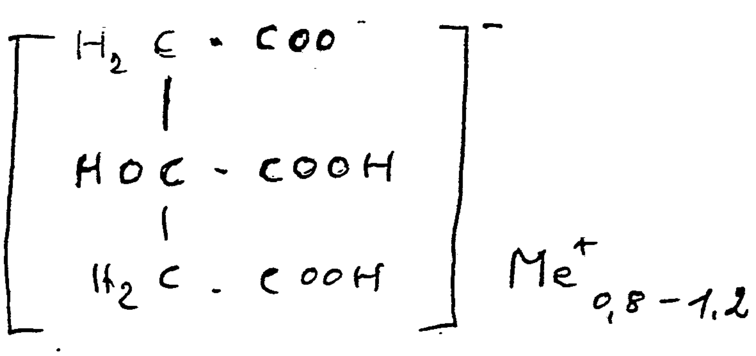

worin $Me^+$ ein Natrium-, Kalium-oder Ammonium-Ion bedeutet.

Durch Anwendung dieser Beobachtung auf hygienische Zellstoffprodukte, wie Kinderwindeln, Erwachsenenwindeln, Krankenunterlagen, Damenbinden oder Slipeinlagen können Produkte mit besonderer Hautverträglichkeit geschaffen werden. Als Beispiel einer besonders vorteilhaften Ausführungsform der Erfindung wird vorgeschlagen, daß die Zellstoff-Flocken des Saugkörpers dieser Produkte mit partiell neutralisierter Zitronensäure der genannten Art imprägniert sind. Allerdings ist es auch möglich, gesondert hergestellte Zellstoffblätter, also beispielsweise Tissueblätter mit partiell neutralisierter Zitronensäure zu imprägnieren und diese als Einlagen in die Produkte einzuarbeiten. Bevorzugt wird es allerdings, die Flocken selbst zu imprägnieren. Die imprägnierte Schicht sollte dabei vorzugsweise 1,0 - 3,0 Gewichtsprozent der partiell neutralisierten Zitronensäure enthalten. Die imprägnierte oder mit Einlagen ausgestattete Schicht kann außer Zellstoff-Flocken auch noch andere Stoffe, z. B. Quellstoffe oder Fasern enthalten.

Als besonders vorteilhaft kommt noch hinzu, daß Produkte dieser Art wirtschaftlich hergestellt werden können, wenn dabei so verfahren wird, wie dies an sich bekannt und üblich ist, indem also zur Herstellung der Zellstoff-Flocken des Saugkissens zunächst von Trockenzellstoff (etwa Blattzellstoff) ausgegangen wird, dieser Trockenzellstoff aber in Abweichung vom Bekannten zunächst mit einer wässrigen Lösung partiell neutralisierter Zitronsäure imprägniert und dann erst zur Flocke verarbeitet wird, worauf die so gewonnenen imprägnierten Zellstoff-Flocken in üblicher Weise zum Fertigartikel weiterverarbeitet werden. So zu verfahren gestattet es, die üblichen und meist schon vorhandenen Produktionsanlagen weiter zu benutzen, wobei lediglich eine Sprühstation für die Applikation einer wässrigen Lösung der partiell neutralisierten Zitronensäure eingeschaltet werden muß. Als vorteilhaft hat es sich erwiesen, wenn für die Applikation der Säure eine Lösung folgender Zusammensetzung verwendet wird:

Wasser: 78,4 Liter
Natronlauge (32 %ig): 9,4 Liter
Zitronensäure-Monohydrat: 36,7 kg

[illegible]bor-und Klinik-Untersuchungen haben gezeigt, daß die erfindungsgemäß ausgerüsteten Produkte [illegible] hautfreundlicher sind und ein beträchtlich verringertes Wachstum von Mikroorganismen (sowohl [illegible] wie auch Pilzen) zeigen als nicht ausgerüsteten Produkte. Die durchgeführten Versuche wurden unter Verwendung von Höschenwindeln an sich bekannten Aufbaues durchgeführt. Die Citrat-imprägnierten Windeln enthielten partiell neutralisierte Zitronensäure im Flockenkissen; die Eintragsmenge betrug ca. 1 %. Die nachfolgende Zusammenstellung zeigt die Ergebnisse der Messungen.

1. Allgemeine Angaben zur Testdurchführung

1.1 Anzahl der untersuchten Kinder : 20
1.2 Geschlecht der Kinder : 14 männliche, 6 weibliche
1.3 Alter der Kinder : 1 - 27 Monate
< 6 Monate : 12 Kinder
> 6 Monate : 8 Kinder

1.4 Beobachtungszeitrum (Anzahl der Kinder)

| | |
|---|---|
| < 2 Wochen | 6 |
| 2 - 4 Wochen | 8 |
| > 4 Wochen | 6 |

1.5 Tragedauer (Fallzahl/Mittelwerte Stunden)

| | Zitrat + | Zitrat - | mittlere Tragedauer (Stunden) |
|---|---|---|---|
| < 4 Stunden | 54 | 14 | 3,0 |
| 4,25 - 8 Std. | 24 | 85 | 5,9 |
| > 8,25 Stunden | 44 | 61 | 11,5 |

1.6 Testmuster Windeln (Anzahl)

| | |
|---|---|
| Zitrat + | 122 Stück |
| Zitrat - | 160 Stück |

| | Zitrat + | Zitrat - |
|---|---|---|
| Windelgröße 1: | - | 81 |
| Windelgröße 2: | 122 | 119 |

1.7 Ausscheidungen (Fallzahl)

| | |
|---|---|
| Urin | 282 |
| Urin und Stuhl | 33 |

1.8 mittlere Ausscheidungen in g (Fallzahl)

| Tragedauer | Zitrat + | Zitrat - |
|---|---|---|
| < 4 Stunden | 54,0 (53) | 53,8 (15) |
| 4,25 - 8 Stunden | 71,9 (24) | 67,7 (108) |
| > 8,25 Stunden | 114,2 (44) | 103,4 (77) |

## 2. Hautauffälligkeiten im Test

| | Zitrat + | Zitrat - |
|---|---|---|
| • Fallzahl | 23 | 35 |
| • Gesamtzahl der im Test untersuchten Windelwechsel | 122 | 160 |
| • Häufigkeit im Test | 18,9 | 21,9 |

| Tragedauer/Fallzahl | Zitrat + | Zitrat - |
|---|---|---|
| < 4 Stunden | 6 | 1 |
| 4,25 - 8 Stunden | 9 | 21 |
| > 8,25 Stunden | 8 | 13 |

## 3. Mittlere PH-Werte in Urinausscheidungen (Anzahl der Proben)

| Tragedauer | Zitrat + | Zitrat - |
|---|---|---|
| < 4 Stunden | 5,98 (54) | 6,51 (14) |
| 4,25 - 8 Stunden | 5,75 (24) | 6,56 (85) |
| > 8,25 Stunden | 6,35 (44) | 7,35 (61) |

## 4. Mittlere aerobe Gesamtkeimzahl ($N \cdot 10^{-8} \cdot ml^{-1}$) - (Anzahl der Proben)

| Tragedauer | Zitrat + | Zitrat - |
|---|---|---|
| < 4 Stunden | 0,12 (93) | 9,28 (15) |
| 4,25 - 8 Stunden | 4,34 (24) | 9,17 (91) |
| > 8,25 Stunden | 15,15 (42) | 11,87 (60) |

## 5. Mittlere aerobe Gesamtkeimzahl ($N \cdot 10^{-8} \cdot ml^{-1}$) - (Anzahl der Proben)

| Tragedauer | Zitrat + | Zitrat - |
|---|---|---|
| < 4 Stunden | 0,13 (53) | 0,53 (15) |
| 4,25 - 8 Stunden | 3,82 (24) | 8,67 (91) |
| > 8,25 Stunden | 9,77 (42) | 10,58 (60) |

6. Candida-Befunde (Fallzahl/Zahl der Proben)

| | | |
|---|---|---|
| Zitrat + | : | 5 |
| Zitrat - | : | 10 |

Mittlere Zahl von Candida-Keimen ($N \cdot 10^{-3} \cdot ml^{-1}$) - (Anzahl der Fälle)

| | | |
|---|---|---|
| Zitrat + | 0,63 | ( 5) |
| Zitrat - | 19,95 | (10) |

7. Mittlere Anzahl von Staphylokokken ($N \cdot 10^{-6} \cdot ml^{-1}$) - (Probenzahl)

| Tragedauer | Zitrat + | Zitrat - |
|---|---|---|
| < 4 Stunden | 0,13 | 0,97 |
| 4,25 - 8 Stunden | 0,10 | 5,60 |
| > 8,25 Stunden | 4,24 | 8,82 |

8. Mittlere Anzahl von Streptokokken ($N \cdot 10^{-6} \cdot ml^{-1}$) - (Probenzahl)

| Tragedauer | Zitrat + | Zitrat - |
|---|---|---|
| < 4 Stunden | 0,07 (52) | 0,91 (15) |
| 4,25 - 8 Stunden | 0,72 (24) | 4,61 (91) |
| > 8,25 Stunden | 2,14 (42) | 6,67 (61) |

9. Mittlere Anzahl von Enterobakterien ($N \cdot 10^{-8} \cdot ml^{-1}$) - (Probenzahl)

| Tragedauer | Zitrat + | Zitrat - |
|---|---|---|
| < 4 Stunden | 0,08 (53) | 9,14 (15) |
| 4,28 - 8 Stunden | 4,63 (24) | 14,29 (91) |
| > 8,25 Stunden | 11,48 (42) | 8,53 (60) |

10. Mittlere Anzahl von Proteuskeimen ($N \cdot 10^{-8} \cdot ml^{-1}$) - (Probenzahl)

| Tragedauer | Zitrat + | Zitrat - |
|---|---|---|
| $<4$ Stunden | 0,17 (53) | 4,60 (15) |
| 4,25 - 8 Stunden | 3,27 (24) | 8,62 (91) |
| $>8{,}25$ Stunden | 11,22 (42) | 12,25 (60) |

11. Mittlere Anzahl von hämolytischen anaroben Keimen ($N \cdot 10^{-8} \cdot ml^{-1}$) - Probenzahl

| Tragedauer | Zitrat + | Zitrat - |
|---|---|---|
| $<4$ Stunden | 0,18 (53) | 4,28 (15) |
| 4,25 - 8 Stunden | 2,62 (24) | 7,96 (91) |
| $>8{,}25$ Stunden | 12,44 (42) | 11,15 (60) |

Weitere Untersuchungsergebnisse gehen aus der nachfolgenden Zusammenstellung hervor:

1. Angaben zum Kinderkollektiv

| Initialen | Alter | Geschlecht | Anzahl untersuchter Windeln Zitrat- | Zitrat+ | Beobachtungszeitraum (Tage) |
|---|---|---|---|---|---|
| RD | 5 W | M | 15 | - | 20 |
| MF | 10 W | M | - | 11 | 7 |
| GN | 3 M | W | 4 | - | 2 |
| MK | 6 M | W | - | 26 | 22 |
| HEM | 11 M | W | 49 | 44 | 157 |
| MJ | 5 M | M | 33 | 31 | 147 |
| LJ | 3,5 J | W | - | 3 | 2 |

2. Tragedauer/Anzahl Windeln

| | Zitrat - | Zitrat + |
|---|---|---|
| $<4$ Stunden | 43 | 64 |
| 4,25 - 8 Stunden | 42 | 41 |
| $>8{,}25$ Stunden | 16 | 9 |

3. Ausscheidungen/Fallzahl

| | Zitrat - | Zitrat + |
|---|---|---|
| Urin | 79 | 96 |
| Urin und Stuhl | 21 | 18 |

4. Mittlere Ausscheidungen Urin in g (Fallzahl)

| | Zitrat - | Zitrat + |
|---|---|---|
| $<$ 4 Stunden | 47,9 (43) | 55,3 (64) |
| 4,25 - 8 Stunden | 66,5 (42) | 54,5 (41) |
| $>$ 8,25 Stunden | 107,3 (16) | 131,1 (9) |

5. Ergebnisse mikrobiologischer Untersuchungen (Fallzahl)

5.1 Mittlere PH-Werte in Urinausscheidungen (Fallzahl)

| | Zitrat - | Zitrat + |
|---|---|---|
| $<$ 4 Stunden | 7,47 (37) | 5,76 (57) |
| 4,25 - 8 Stunden | 7,68 (42) | 6,40 (39) |
| $>$ 8,25 Stunden | 8,64 (16) | 8,20 (9) |

5.2 Mittlere aerobe Gesamtkeimzahl ($N \cdot 10^{-8} \cdot ml^{-1}$) (Anzahl Windeln)

| | Zitrat - | Zitrat + |
|---|---|---|
| $<$ 4 Stunden | 0,35 (43) | 0,07 (64) |
| 4,25 - 8 Stunden | 3,05 (42) | 0,67 (41) |
| $>$ 8,25 Stunden | 10,83 (16) | 17,4 (9) |

5.3 Candida-Befunde

5.3.1 Fallzahl (Anzahl untersuchter Windeln)

| Zitrat - | Zitrat + |
|---|---|
| 62 (101) | 48 (111) |

5.3.2 Mittlere Keimzahl ($N \cdot 10^{-3} \cdot ml^{-1}$) (Anzahl Windeln)

| | Zitrat - | Zitrat + |
|---|---|---|
| | 20,5 (62) | 7,1 (48) |

5.4 Mittlere Anzahl Staphylokokken ($N \cdot 10^{-6} \cdot ml^{-1}$) (Anzahl Windeln)

| | Zitrat - | Zitrat + |
|---|---|---|
| < 4 Stunden | 0,024 (43) | 0,012 (64) |
| 4,25 - 8 Stunden | 0,47 (41) | 0,016 (41) |
| > 8,25 Stunden | 0,11 (16) | 0,055 ( 9) |

5.5 Mittlere Anzahl Enterobakterien ($N \cdot 10^{-8} \cdot ml^{-1}$) (Anzahl Windeln)

| | Zitrat - | Zitrat + |
|---|---|---|
| < 4 Stunden | 0,091 (43) | 0,087 (64) |
| 4,25 - 8 Stunden | 3,15 (42) | 0,428 (41) |
| > 8,25 Stunden | 7,98 (16) | 5,795 ( 9) |

5.6 Mittlere Anzahl Proteus ($N \cdot 10^{-8} \cdot ml^{-1}$) (Anzahl Windeln)

| | Zitrat - | Zitrat + |
|---|---|---|
| < 4 Stunden | 0,085 (42) | 0,055 (64) |
| 4,25 - 8 Stunden | 2,72 (42) | 0,401 (41) |
| > 8,25 Stunden | 9,99 (16) | 17,36 ( 9) |

| 6. Hautbefunde | Zitrat - | Zitrat + |
|---|---|---|
| 6.1 beobachtete Hautauffälligkeiten (Anzahl untersuchter Windeln) | 59 (86) | 41 (98) |
| 6.2 Häufigkeit | 68,6 % | 41,8 % |

7. Ergebnisse

Die Keimzahlreduktion (Quotient: Keimzahl Zit-/Keimzahl Zit+ Proben) bei Zitratwindeln im Vergleich zu den Nullproben beträgt für mittlere Tragezeiten (4,25 - 8 Stunden):

aerobe Gesamtkeime : 4,6
Candida sp. : 2,9
Staphylokokken sp. : 29,4
Enterobakterien sp. : 7.4
Proteus sp. : 6,8

Die Ergebnisse für Reduktionsraten und Keimzahlniveaus (GKZ, Enterobakterien, Proteus, Candida) sind vergleichbar mit früheren Ergebnissen im Panaltest. Starke Abweichungen ergeben sich insbesondere bei den Keimzahlniveaus für Staphylokokken (bei Zit-Mustern im Test mehr als eine Größenordnung erniedrigt) und bei der Häufigkeit des Auftretens von Haut-und Candida-Befunden (im Test 5 - 10-fach erhöht).

## Ansprüche

1. Hygienisches Zellstoff-Produkt, wie Windel, Krankenunterlage, Damenbinde oder Slipeinlage, bestehend aus einem Zellstoff-Flocken enthaltenden Saugkissen, einer flüssigkeitsdurchlässigen oberen Abdeckung sowie gegebenenfalls einer flüssigkeitsdichten unteren Abdeckung oder Einlage,
dadurch gekennzeichnet,
daß wenigstens eine Schicht des Produktes mit partiell neutralisierter Zitronensäure der Zusammensetzung

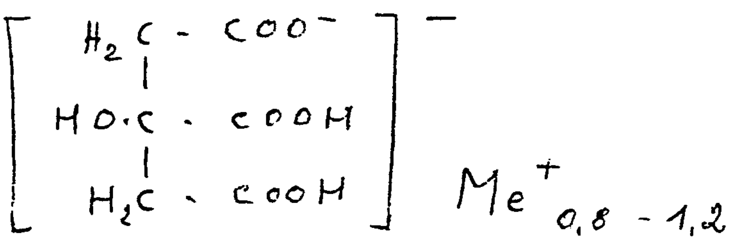

imprägniert ist, worin $Me^+$ ein Natrium-, Kalium-oder Ammonium-Ion bedeutet.

2. Hygienishes Zellstoff-Produkt nach Anspruch 1,
dadurch gekennzeichnet,
daß die Zellstoff-Flocken des Saugkörpers mit partiell neutralisierter Zitronensäure imprägniert sind.

3. Hygienisches Zellstoff-Produkt nach Anspruch 1 oder 2,
dadurch gekennzeichnet,
daß die imprägnierte Schicht 1,0 - 3,0 Gewichtsprozent partiell neutralisierter Zitronensäure enthält.

4. Hygienisches Zellstoff-Produkt nach Anspruch 1, 2 oder 3,
dadurch gekennzeichnet,
daß der Saugkörper zusätzlich Quellstoffe enthält.

5. Verfahren zum Herstellen eines mit partiell neutralisierter Zitronensäure imprägnierten Zellstoff-Produktes nach einem der Ansprüche 1 bis 4,
dadurch gekennzeichnet,
daß zur Herstellung der Zellstoff-Flocken des Saugkissens in an sich bekannter Weise von Trockenzellstoff ausgegangen, dieser mit einer wässrigen Lösung partiell neutralisierter Zitronensäure imprägniert und sodann zerflockt wird, worauf die so gewonnenen Zellstoff-Flocken in üblicher Weise zum Fertigartikel weiterverarbeitet werden.

6. Verfahren nach Anspruch 4,
dadurch gekennzeichnet,
daß eine Lösung folgender Zusammensetzung verwendet wird:

Wasser: 78,4 Liter
Natronlauge (32 %ig): 9,4 Liter
Zitronensäure-Monohydrat: 36,7 kg